# EUROPEAN PATENT SPECIFICATION

(11) **EP 0 333 282 B1**
(45) Date of publication and mention of the grant of the patent: **09.06.1993**
(21) Application number: 89200631.3
(22) Date of filing: 10.03.1989
(51) Int. Cl.: C07C 233/81, C07C 231/00, A01N 37/46

(54) **Preparation of optically active N-substituted alanine herbicides**
Herstellung von optisch aktiven N-substituierten Alaninherbiciden
Préparation d'herbicides optiquement actifs du type alanine N-substituée

(30) Priority: 16.03.1988 GB 8806223
(43) Date of publication of application: 20.09.1989
(73) Proprietor: SHELL INTERNATIONALE RESEARCH MAATSCHAPPIJ B.V., 2596 HR Den Haag (NL)
(72) Inventor: Miedema, Alle, NL-3161 LG Rhoon (NL)

(56) References cited:
- DE-A- 1 768 173
- DE-A- 2 008 592
- DE-A- 2 460 691
- GB-A- 1 563 201
- US-A- 4 354 032

## Description

This invention relates to the preparation of optically active N-substituted alanine herbicides.

Our UK Patent Specification No. 1 563 201 describes and claims a process for the preparation of certain optically active N-substituted alanine compounds of the general formula I possessing the R-configuration at C²: 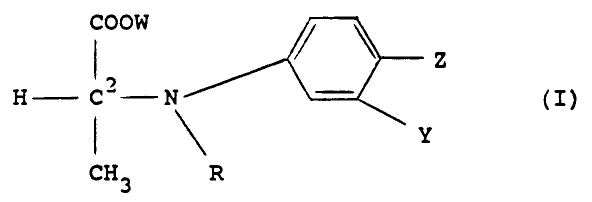
wherein
Y and Z each independently represents a hydrogen, chlorine or fluorine atom;
W represents a hydrogen atom, an alkyl, haloalkyl, alkenyl, cycloalkyl, aryl, alkaryl or aralkyl group, or a group of the general formula -N=CR¹R² in which R¹ and R² each independently represents a hydrogen atom, or an alkyl, aryl, aralkyl, alkaryl or alkenyl group, or R¹ and R² together represent an alkylene group which is optionally interrupted by one or more hetero atoms; and
R represents a hydrogen atom or a benzoyl or thiobenzoyl group.

Preferred compounds are those in which R represents a benzoyl group and such benzoylated compounds have found wide use as selective herbicides in cereal crops. Those isomers having the R-configuration at carbon atom 2 have been found to have better herbicidal activity than the racemic mixture, thus allowing less herbicide to be used with equivalent effect on weeds in a crop.

In the preparation of the optically active compounds of the general formula I defined above, an optically active derivative of lactic acid is reacted with an aniline to give a compound of the general formula I in which R represents a hydrogen atom. The benzoylated derivatives are then prepared by reaction with a benzoyl halide, suitably under refluxing conditions in a suitable solvent.

It has been found that while the reaction of the lactic acid and the aniline can be carried out even on a large scale with maintenance of high optical purity, the benzoylation reaction, when carried out on a scale larger than that encountered in the laboratory, results in a substantial loss of optical purity.

The present invention seeks to overcome this problem.

We have found that, surprisingly, the inclusion of a tertiary amine allows one to obtain a benzoylated product in which there has been a minimal loss of optical purity, even on a large-scale operation.

Accordingly, the present invention provides a process for the preparation of an optically active compound of general formula I, as defined above, in which R represents a benzoyl group, which comprises benzoylating a starting compound of the general formula I in which R represents a hydrogen atom and possessing the R-configuration at C², with a benzoylating agent in the presence of a solvent and a tertiary amine.

Suitable examples of tertiary amines include tri(C₁-C₆)alkylamines, for example tributylamine and triethylamine, and N-heterocyclic tertiary amines, for example pyridine and quinoline. The preferred tertiary amine is triethylamine.

The amount of tertiary amine employed is preferably in excess of the equivalent molar amount required to react with the hydrogen halide released in the benzoylation reaction; for example in the range of from 1 to 1.5 moles of tertiary amine per mole of starting compound, suitably about 1.25 moles per mole of starting compound.

Suitable examples of solvents employed for the benzoylation include, especially, aromatic solvents such as benzene, toluene and xylene. Preferably, the solvent used is toluene. The process of the invention may conveniently be carried out at the reflux temperature of the solvent employed.

The benzoylating agent is suitably a benzoyl halide, usually benzoyl chloride. An example of an alternative benzoylating agent that may be used, is benzoyl fluoride. The benzoylating agent and the starting compound are preferably employed in substantially equimolar amounts or with excess benzoylating agent, for example from 1 to 1.5 moles of benzoylating agent per mole of starting compound, suitably about 1.2 moles.

Preferred starting compounds of general formula I in which R represents a hydrogen atom are those in which W represents a methyl or isopropyl group, Z represents a fluorine atom and Y represents a chlorine atom. The starting compounds of general formula I in which R represents a hydrogen atom may be prepared as described in UK Patent Specification No. 1 563 201.

The following Examples illustrate the invention.

### Example 1

Benzoyl chloride (2838 kg; 20.2k moles) was dosed over a period of 3 hours into a solution (75°C) of toluene (13670 l) containing feedstock, designated A, of R-(+)-2-(3-chloro-4-fluoroanilino)-propionic acid isopropyl ester (4325 kg; 16.7k mole) and triethylamine (2065 kg; 20.4k mole) in a reactor of volume 25m³. After a post reaction period of 3 hours at 75°C, the product R(-)-2-(N-benzoyl-3-chloro-4-fluoroanilino)-propionic acid isopropyl ester was recovered in a molar yield of 78% after crystallisation. The work up procedure after benzoylation consisted of neutralisation of excess triethylamine by dosing with anhydrous hydrochloric acid, addition of a mixture of toluene and water, phase separation, bicarbonate and water washing and removal of toluene.

The benzoylation was repeated with a further batch of feedstock A and with an alternative feedstock, feedstock B, having a different initial chemical and optical purity. The results, expressed in percentage form, are shown in Table 1 below.

**Table 1**

| Feedstock | | Feedstock | | Product | |
|---|---|---|---|---|---|
| | | Chemical Purity | Optical Purity | Chemical Purity | Optical Purity |
| A | Batch 1 | 92.0 | 84.6 | 79.4 | 74.0 |
| | Batch 2 | 92.0 | 84.6 | 78.7 | 74.7 |
| B | | 81.1 | 79.0 | 73.4 | 70.1 |

### Example 2 (Comparative)

The procedure described in Example 1 above was repeated with a feedstock, designated C, but omitting the triethylamine and adding the benzoyl chloride at reflux temperatures over a period of 12.5 hours. Measurements of chemical and optical purity were taken immediately after addition and also after post reaction times of 0.75 and 3.0 hours.

The benzoylation was repeated with a further feedstock, feedstock D, which was added over a dosing period of 4 hours under reflux at a pressure of 1.2 bar (1.2x10⁵ Pa).

The results are shown as percentages in Table 2 below.

**Table 2**

| Feedstock | Feedstock | | Product | |
|---|---|---|---|---|
| | Chemical Purity | Optical Purity | Chemical Purity | Optical Purity |
| C | 88.3 | 85.5 | 85.6 (1) | 53 (1) |
| | | | 88.1 (2) | 49.6 (2) |
| | | | 81.2 (3) | 44.3 (3) |
| D | 90.8 | 89.1 | 87.6 | 69.2 |
| Notes: (1) Directly after benzoyl chloride addition. | | | | |
| (2) After post reaction of 0.75 hours. | | | | |
| (3) After post reaction of 3 hours. | | | | |

### Example 3

Experiments 1 and 2 were carried out on a single feedstock, designated E, of R-(+)-2-(3-chloro-4-fluoroanilino)-propionic acid isopropyl ester in toluene having a chemical purity (based on the isopropyl ester) of 45.3% (m/m) and an optical purity of 42.3%.

Experiment 1 was carried out with the addition of triethylamine while Experiment 2 was carried out without triethylamine.

Experiment 1 was carried out under the normal plant conditions of temperature and pressure that would be applicable for the process on a large-scale operation.

Experiment 2 was carried out at a higher temperature and pressure than Experiment 1 so as to simulate on a laboratory scale the plant conditions that would apply in the absence of the tertiary amine. When employing no tertiary amine the static height of the liquid under normal plant conditions is increased and gives rise to loss of optical purity. The higher pressure of Experiment 2 simulates the pressure that would be generated in the plant by the static height of liquid; this higher pressure prevents boiling at the bottom of the reactor and a higher temperature is thus required for reflux to occur.

The quantities of reactants fed into the reactor and withdrawn therefrom and the chemical purity and yield of product are given in Table 3. In the Table "TEAM" stands for triethylamine.

Working up of the product after benzoylation was carried out as described for Example 1, except that, of course, for Experiment 2 no neutralisation of excess triethylamine was necessary.

**Table 3**

| | Experiment 1 | Experiment 2 |
|---|---|---|
| In | | |
| ester/toluene g/mol | 200.3/0.35 | 200.8/0.35 |
| TEAM (99%) g/mol | 45 /0.45 | - |
| Benzoyl chloride (99.5%) g/mol | 59.7/0.42 | 60.3/0.427 |

| Out | | |
|---|---|---|
| Benzoylated ester/toluene g | 301.8 | 298.3 |
| Crude benzoylated ester g | 131.1 | 124.3 |
| Chemical purity %(m/m) | 90.5 | 90.7 |
| Optical purity % | 81 | 57 |
| Optical retention % | 91.6 | 74.5 |

| Chemical yield | | |
|---|---|---|
| benzoylated ester/non benzoylated ester | 93.3 | 88.5 |

| Reaction Conditions | | |
|---|---|---|
| Temperature (°C) | 75 | 115 |

| Pressure | | |
|---|---|---|
| (bar) | 1 | 1.6 |
| (Pa) | 10⁵ | 1.6x10⁵ |

## Claims

1. A process for the preparation of an optically active compound of the general formula I 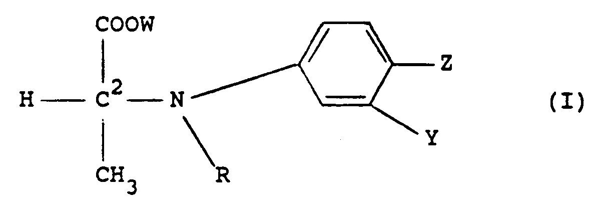 wherein
Y and Z each independently represents a hydrogen, chlorine or fluorine atom;
W represents a hydrogen atom, an alkyl, haloalkyl, alkenyl, cycloalkyl, aryl, alkaryl or aralkyl group, or a group of the general formula -N=CR¹R² in which R¹ and R² each independently represents a hydrogen atom or an alkyl, aryl, aralkyl, alkaryl or alkenyl group, or R¹ and R² together represent an alkylene group which is optionally interupted by one or more hetero atoms; and
R represents a benzoyl group,
which comprises benzoylating a starting compound of the general formula I, in which R represents a hydrogen atom and possessing the R-configuration at C² , with a benzoylating agent in the presence of a solvent and a tertiary amine.

2. A process as claimed in claim 1, wherein the tertiary amine is triethylamine.

3. A process as claimed in claim 1 or claim 2, wherein the solvent is toluene.

4. A process as claimed in any one of claims 1 to 3, wherein the tertiary amine is present in an amount in the range of from 1 to 1.5 moles of amine per mole of the starting compound of general formula I.

5. A process as claimed in any one of claims 1 to 4, wherein the benzoylating agent is benzoyl chloride.

6. A process as claimed in any one of claims 1 to 5, wherein the starting compound is of general formula I in which R represents a hydrogen atom, W represents a methyl or isopropyl group, Z represents a fluorine atom and Y represents a chlorine atom.

7. A process as claimed in claim 6, wherein W represents an isopropyl group.

## Patentansprüche

1. Verfahren zur Herstellung einer optisch aktiven Verbindung der allgemeinen Formel I 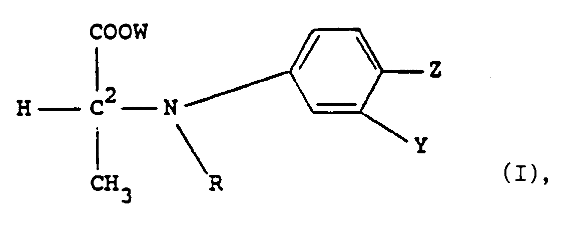 worin
Y und Z jeweils unabhängig voneinander ein Wasserstoff-, Chlor- oder Fluoratom darstellen;
W ein Wasserstoffatom, eine Alkyl-, Halogenalkyl-, Alkenyl-, Cycloalkyl-, Aryl-, Alkaryl- oder Aralkylgruppe oder eine Gruppe der allgemeinen Formel -N=CR¹R² bedeutet, worin R¹ und R² jeweils unabhängig voneinander für ein Wasserstoffatom oder eine Alkyl-, Aryl-, Aralkyl-, Alkaryl- oder Alkenylgruppe bedeuten oder R¹ und R² gemeinsam eine Alkylengruppe darstellen, die gegebenenfalls durch ein oder mehrere Heteroatome unterbrochen ist; und
R für eine Benzoylgruppe steht,
welches Verfahren ein Benzoylieren einer Ausgangsverbindung der allgemeinen Formel I, worin R ein Wasserstoffatom darstellt und welche Ausgangsverbindung am C²-Kohlenstoffatom die R-Konfiguration aufweist, mit eine Benzoylierungsmittel in Anwesenheit eines Lösungsmittels und eines tertiären Amins umfaßt.

2. Verfahren nach Anspruch 1, worin das tertiäre Amin Triethylamin ist.

3. Verfahren nach Anspruch 1 oder 2, worin das Lösungsmittel Toluol ist.

4. Verfahren nach einem der Ansprüche 1 bis 3, worin das tertiäre Amin in einer Menge im Bereich von 1 bis 1,5 Mol Amin je Mol Ausgangsverbindung der allgemeinen Formel I vorliegt.

5. Verfahren nach einem der Ansprüche 1 bis 4, worin das Benzoylierungsmittel Benzyolchlorid ist.

6. Verfahren nach einem der Ansprüche 1 bis 5, worin die Ausgangsverbindung der allgemeinen Formel I entspricht, worin R ein Wasserstoffatom darstellt, W eine Methyl- oder Isopropylgruppe bedeutet, Z ein Fluoratom darstellt und Y ein Chloratom bedeutet.

7. Verfahren anch Anspruch 6, worin W eine Isopropylgruppe darstellt.

## Revendications

1. Un procédé pour la préparation d'un composé optiquement actif de la formule générale I 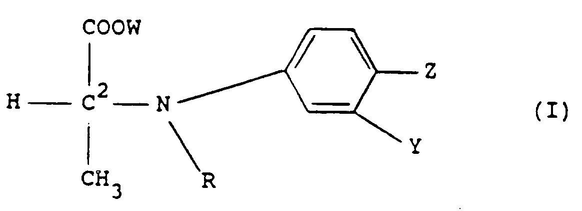 dans laquelle
Y et Z représentent chacun indépendamment un atome d'hydrogène, de chlore ou de fluor;
W représente un atome d'hydrogène, un groupe alkyle, haloalkyle, alcényle, cycloalkyle, aryle, alkaryle ou aralkyle, ou bien un groupe de la formule générale - N=CR¹R² dans laquelle R¹ et R² représentent chacun indépendamment un atome d'hydrogène ou un groupe alkyle, aryle, aralkyle, alkaryle ou alcényle, ou bien R¹ et R² représentent conjointement un groupe alcylène qui est facultativement interrompu par un ou plus d'un hétéro-atome; et
R représente un groupe benzoyle, procédé
dans lequel on soumet à une benzoylation un composé de départ de la formule générale I, dans laquelle R représente un atome d'hydrogène et possédant une configuration R en C², avec un agent de benzoylation en présence d'un solvant et d'une amine tertiaire.

2. Un procédé tel que revendiqué dans la revendication 1, dans lequel l'amine tertiaire est la triéthylamine.

3. Un procédé tel que revendiqué dans la revendication 1 ou la revendication 2, dans lequel le solvant est le toluène.

4. Un procédé tel que revendiqué dans l'une quelconque des revendications 1 à 3, dans lequel l'amine tertiaire est présente en une quantité dans la gamme de 1 à 1,5 moles d'amine par mole de composé de départ de formule générale I.

5. Un procédé tel que revendiqué dans l'une quelconque des revendications 1 à 4, dans lequel l'agent de benzoylation est le chlorure de benzoyle.

6. Un procédé tel que revendiqué dans l'une quelconque des revendications 1 à 5, dans lequel le composé de départ correspond à la formule générale I dans laquelle R représente un atome d'hydrogène, W représente un groupe méthyle ou isopropyle, Z représente un atome de fluor et Y représente un atome de chlore.

7. Un procédé tel que revendiqué dans la revendication 6, dans lequel W représente un groupe isopropyle.
